Europäisches Patentamt

European Patent Office

Office européen des brevets

(19)

(11) Veröffentlichungsnummer: **0 465 816 A1**

## (12) EUROPÄISCHE PATENTANMELDUNG

(21) Anmeldenummer: **91108853.2**

(22) Anmeldetag: **29.05.91**

(51) Int. Cl.5: **A61F 2/52**

(30) Priorität: **11.07.90 DE 9010426 U**

(43) Veröffentlichungstag der Anmeldung:
**15.01.92 Patentblatt 92/03**

(84) Benannte Vertragsstaaten:
**AT DE ES FR GB IT NL SE**

(71) Anmelder: **AMOENA**
**Medizin-Orthopädie-Technik GmbH**
**Kapellenweg 36**

**W-8201 Raubling(DE)**

(72) Erfinder: **Mulligan, Elisabeth**
**Tulpenweg 3**
**W-8201 Söllhuben(DE)**

(74) Vertreter: **Laufhütte, Dieter, Dipl.-Ing., Dr.-Ing.**
**Lorenz-Seidler-Gossel et al**
**Widenmayerstrasse 23**
**W-8000 München 22(DE)**

(54) **Brustprothese.**

(57) Eine Brustprothese (1) besteht aus einem schalenförmigen Körper (2) aus einer weich-elastisch eingestellten additionsvernetzenden Zweikomponenten-Silikonkautschuk-Masse, der in Kunststoffolien (3,4) eingeschweißt ist, die dessen Außenseite und dessen Innenseite abdecken. Zur Lösung der Aufgabe, eine symmetrische Brustprothese (1) dieser Art derart weiterzuentwickeln, daß die linke und die rechte Brustprothese (1) bei identischer Form der Form der natürlichen Brust in guter Weise angepaßt sind, wird der schalenförmige Körper (2) derart geformt, daß er in Draufsicht im wesentlichen die Form eines zu seiner Höhe (Y-Achse) symmetrischen gleichschenkligen Dreicks besitzt.

FIG. 1

EP 0 465 816 A1

Die Erfindung betrifft eine Brustprothese, bestehend aus einem schalenförmigen Körper aus einer weich-elastisch eingestellten additionsvernetzenden Zweikomponenten-Silikonkautschuk-Masse, der in Kunststoffolien eingeschweißt ist, die dessen Außenseite und dessen Innenseite abdecken.

Brustprothesen dieser Art sind beispielsweise aus der DE-AS 26 05 148 und der EP-OS 320 590 bekannt. Diese bekannten Brustprothesen werden zu ihrer Halterung in Büstenhalter eingelegt und weisen eine unsymmetrische Form auf, da sie in Richtung auf die Achselhöhlen verlängert sind, um einen möglichst übergangslosen Anschluß an den Oberkörper zu gewährleisten.

Unsymmetrische Brustprothesen lassen sich grundsätzlich gut der natürlichen Brustform nachbilden, weisen aber den Nachteil auf, daß linke und rechte Prothesen benötigt werden, was schon wegen der unterschiedlichen zu ihrer Herstellung benötigten Formen einen erhöhten Herstellungsaufwand bedeutet.

Aufgabe der Erfindung ist es daher, eine symmetrische Brustprothese der eingangs angegebenen Art in der Weise zu schaffen, daß die linke und die rechte Brustprothese bei identischer Form der Form der natürlichen Brust in guter Weise angepaßt sind.

Erfindungsgemäß wird diese Aufgabe bei einer Brustprothese der gattungsgemäßen Art dadurch gelöst, daß der Körper in Draufsicht im wesentlichen die Form eines zu seiner Höhe symmetrischen gleichschenkeligen Dreiecks besitzt. Durch die dreiecksähnliche Grundform der Brustprothese ist es möglich, den Brustansatz so hoch wie erforderlich auszubilden, da die Prothese sowohl in die linke als auch in die rechte Schale des Büstenhalters eingelegt wird. Die Länge der Prothese in Querrichtung ist etwas kürzer als bei bekannten asymmetrischen Prothesen, was zur Folge hat, daß die erfindungsgemäße Prothese nicht soweit in Richtung der Achselhöhle reicht. Durch die erfindungsgemäße symmetrische Prothese kann daher nicht soviel wegoperiertes Lymphdrüsengewebe ausgeglichen werden, wie dies durch die sich in Richtung der Achselhöhle erstreckenden Lappen von unsymmetrischen Prothesen möglich ist. Dies ist jedoch kein Nachteil, da bei den heutigen Operationsmethoden versucht wird, nur noch das Brustgewebe zu entfernen und soviel Lymphdrüsengewebe wie möglich zu erhalten.

Eine symmetrische Brustprothese verhältnismäßig komplizierter Form ist aus dem DE-GM 89 02 304 bekannt. Diese Brustprothese ist symmetrisch zu einer in ihrer Tragestellung horizontalen Schnittebene ausgebildet, wobei sie mit einem sich verjüngenden, in Richtung der Achselhöhle verlaufenden Lappen versehen ist.

Vorzugsweise sind bei der erfindungsgemäßen Brustprothese die Ecken des Dreiecks abgerundet. Dabei kann die Basis des Dreiecks konvex bogenförmig gekrümmt sein.

Die Symmetrie der erfindungsgemäßen Brustprothese ist über deren gesamten Höhe im wesentlichen dadurch verwirklicht, daß zu der die Höhe des Dreiecks enthaltenden Längsmittelebene rechtwinkelig verlaufende Querschnitte durch den Körper im wesentlichen symmetrisch zur Längsmittelebene sind.

In weiterer Ausgestaltung der Erfindung ist vorgesehen, daß der Körper in seinem durch die Längsmittelebene verlaufenden Querschnitt etwa sichelförmig ausgebildet ist und daß der Querschnitt ausgehend von einer durch dessen Scheitelbereich gezogenen Linie einen zur Dreiecksspitze verlaufenden, im Durchmesser kontinuierlich abnehmenden längeren oberen Schenkel und einen kürzeren unteren Schenkel aufweist, dessen innere Kontur sinuslinienförmig gekrümmt ist. Auf diese Weise vermag die Brustprothese in optimaler Weise die natürliche Brustform zu simulieren.

Ein Ausführungsbeispiel der Erfindung wird nachstehend anhand der Zeichnung näher erläutert. In dieser zeigt

Fig. 1 eine Draufsicht auf die erfindungsgemäße Brustprothese,

Fig. 2 einen Längsschnitt durch die Brustprothese nach Fig. 1 in der Symmetrieebene (Y-Achse),

Fig. 3 einen Querschnitt durch die Brustprothese längs der Linie III-III in Fig. 1 (X-Achse) und

Fig. 4 eine Rückansicht eines Büstenhalters mit einer in ein Büstenhalterkörbchen angelegten Brustprothese.

Die Brustprothese 1 besteht aus einem schalenförmigen Körper 2 aus einer weich-elastisch eingestellten additionsvernetzenden Zwei-Komponenten-Silikonkautschuk-Masse, dessen Außenseite durch eine Polyurethanfolie 3 und desse Innenseite durch eine Polyurethanfolie 4 abgedeckt sind, die längs ihres gemeinsamen Umfangrandes 5 durch eine umlaufende Schweißnaht miteinander verschweißt sind.

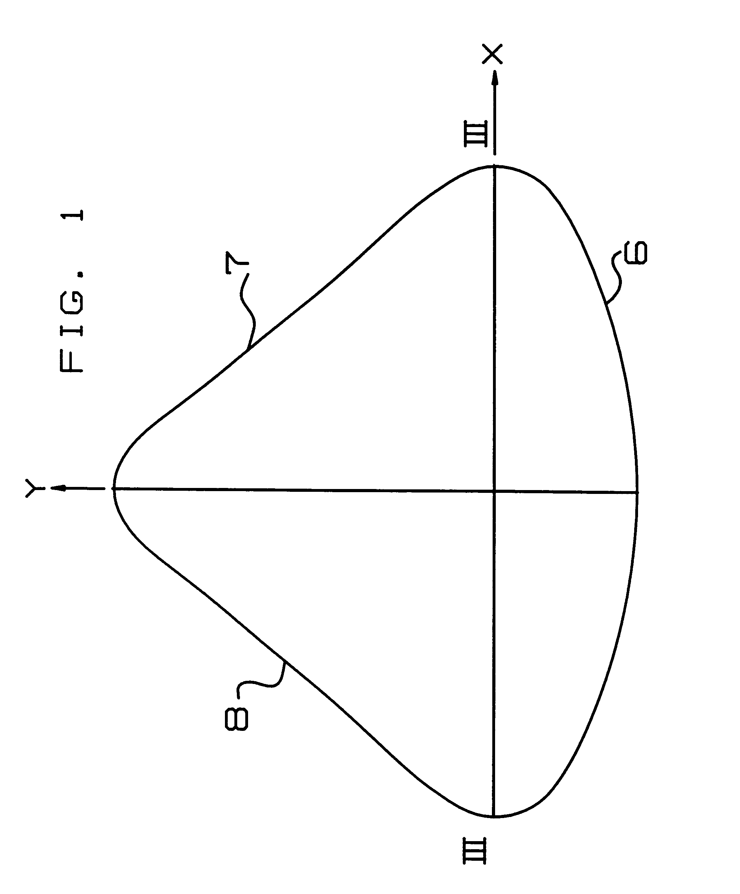

In der aus Fig. 1 ersichtlichen Draufsicht weist die Brustprothese grundsätzlich die Form eines gleichschenkeligen Dreiecks auf, dessen Ecken in der dargestellten Weise abgerundet sind. Die Basis des gleichschenkeligen Dreiecks ist etwa kreisbogenförmig entsprechend der Kurvenlinie 6 konvex gekrümmt. Die Schenkel 7, 8 des Dreiecks schließen einen Winkel von etwa 80° ein.

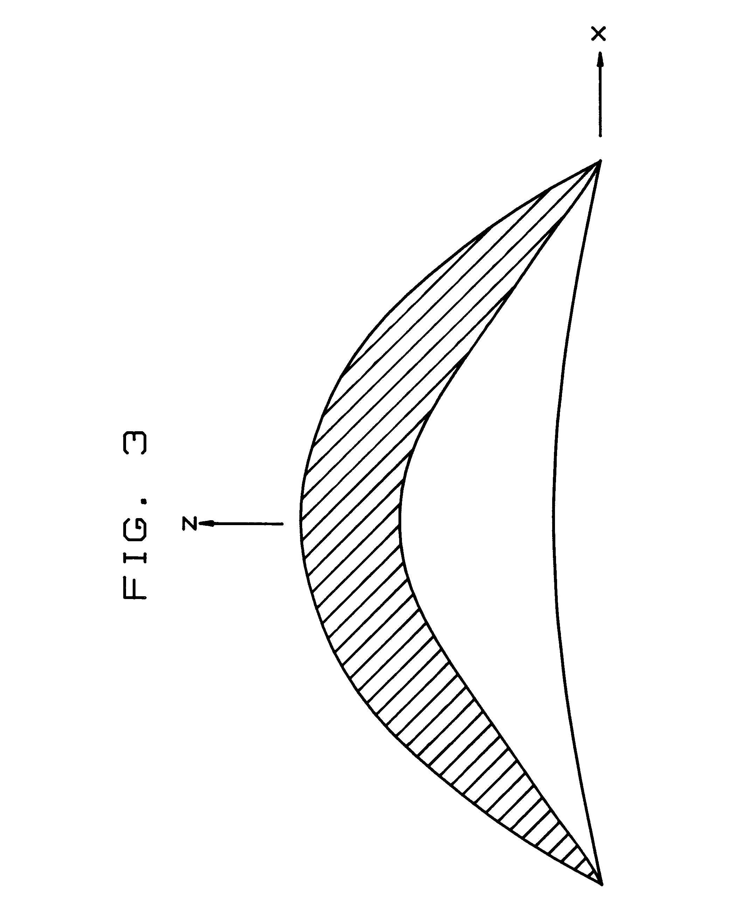

Aus Fig. 3 ist ein Querschnitt längs der Linie III-III in Fig. 1 durch die Brustprothese ersichtlich. Diese Schnittlinie fällt mit der in Fig. 1 eingezeichneten X-Achse zusammen. Der Schnitt gem. Fig. 3 sowie alle weiteren parallel zu der X-Achse ausgeführten Schnitte sind im wesentlichen zu der durch die mit der Y-Achse zusammenfallenden Höhe des Dreiecks verlaufenden Mittelebene symmetrisch.

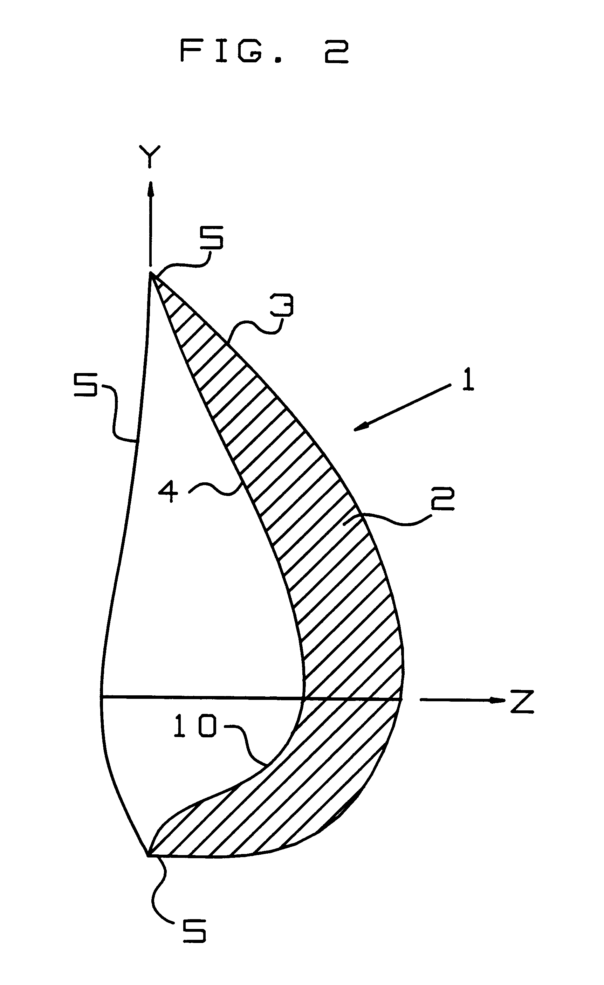

Der durch die Mittelebene der Prothese verlaufende Querschnitt ist aus Fig. 2 ersichtlich. Dieser Querschnitt ist ebenfalls grundsätzlich sichelförmig und besteht ausgehend von einer durch den Scheitel des Profils geführten Linie, die etwa mit der Z-Achse in Fig. 2 zusammenfällt, aus einem oberen längeren Schenkel, dessen Durchmesser sich kontinuierlich zu der Spitze des Dreiecks hin verringert, und aus einem kürzeren unteren Schenkel, dessen innere Kontur 10 sinusförmig ausgebildet ist.

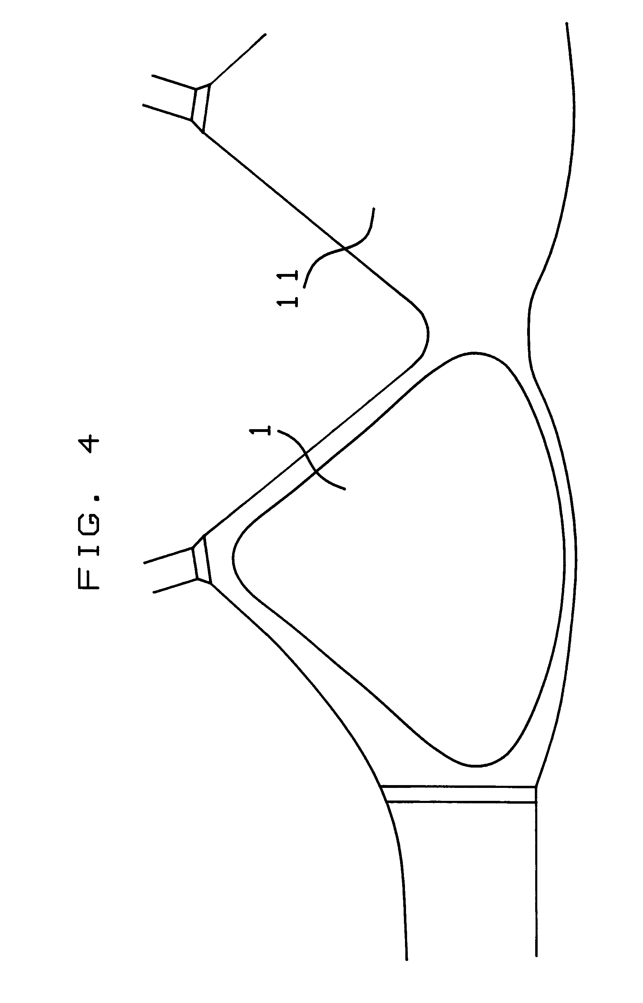

In Fig. 4 ist ein Büstenhalter 11 angedeutet, in dessen linkes Körbchen eine Brustprothese 1 eingelegt ist, die nur durch ihre Umfangsform angedeutet ist.

## Patentansprüche

1. Brustprothese, bestehend aus einem schalenförmigen Körper aus einer weich-elastisch eingestellten additionsververnetzenden Zwei-Komponenten-Silikonkautschuk-Masse, der in Kunststoffolien eingeschweißt ist, die dessen Außenseite und dessen Innenseite abdecken,

   **dadurch gekennzeichnet,**

   daß der Körper (2) in Draufsicht (Fig. 1) im wesentlichen die Form eines zu seiner Höhe (Y-Achse) symmetrischen gleichschenkeligen Dreiecks besitzt.

2. Brustprothese nach Anspruch 1, dadurch gekennzeichent, daß die Ecken des Dreiecks abgerundet sind.

3. Brustprothese nach Anspruch 2 oder 3, dadurch gekennzeichnet, daß die Basis (6) des Dreiecks konvex bogenförmig gekrümmt ist.

4. Brustprothese nach einem der Ansprüche 1 bis 3, dadurch gekennzeichnet, daß zu der die Höhe des Dreiecks enthaltenen Längsmittelebene rechtwinkelig verlaufende Querschnitte durch den Körper (1) im wesentlichen symmetrisch zu dieser Längsmittelebene sind.

5. Brustprothese nach einem der Ansprüche 1 bis 4, dadurch gekennzeichnet, daß der Körper (2) in seinem durch die Längsmittelebene verlaufenden Querschnitt etwa sichelförmig ausgebildet ist und daß der Querschnitt ausgehend von einer durch dessen Scheitelbereich gezogenen Linie einen zur Dreiecksspitze hin verlaufenden in seinem Durchmesser kontinuierlich abnehmenden längeren oberen Schenkel und einen kürzeren unteren Schenkel aufweist, dessen innere Kontur (10) sinuslinienförmig gekrümmt ist.

6. Brustprothese nach einem der Ansprüche 1 bis 5, dadurch gekennzeichnet, daß die Schenkel des Dreiecks einen Winkel von etwa 70° bis 90° und vorzugsweise von etwa 80° einschließen.

FIG. 1

# FIG. 2

# FIG. 3

FIG. 4

# EUROPÄISCHER RECHERCHENBERICHT

Nummer der Anmeldung

**EP 91 10 8853**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | DE-U-8 902 304 (EBERL)<br>* Insgesamt *<br>– – – | 1-6 | A 61 F 2/52 |
| Y | US-A-4 100 621 (ETTIPIO)<br>* Figur 2; Zusammenfassung *<br>– – – | 1-6 | |
| A,D | DE-A-2 605 148 (RECHENBERG)<br>* Ansprüche; Figuren *<br>– – – | 1-5 | |
| P,X | DE-U-9 010 426 (AMOENA)<br>* Insgesamt *<br>– – – | 1-6 | |
| A | US-A-4 828 559 (GREENBERG)<br>– – – | | |
| A | US-A-3 807 412 (CONNELLY)<br>– – – – – | | |

|  |
|---|
| **RECHERCHIERTE SACHGEBIETE (Int. Cl.5)** |
| A 61 F |

Der vorliegende Recherchenbericht wurde für alle Patentansprüche erstellt

| Recherchenort | Abschlußdatum der Recherche | Prüfer |
|---|---|---|
| Den Haag | 18 September 91 | KLEIN C. |